# EUROPEAN PATENT APPLICATION

(11) **EP 0 693 289 A2**
(43) Date of publication of application: **24.01.1996**
(21) Application number: 95303731.4
(22) Date of filing: 31.05.1995
(51) Int. Cl.: A61L 2/24

(54) **Sterilizer apparatus**

(30) Priority: 22.07.1994 GB 9414795
(71) Applicant: Smiths Industries Public Limited Company, London, NW11 8DS (GB)
(72) Inventor: Hannant, Keith, Rustington, West Sussex BN16 2QN (GB)
(74) Representative: Flint, Jonathan McNeill

(57) **Abstract**

A sterilizer has an alarm 2 for indicating the end of a sterilization cycle and any fault. The alarm 2 is located remote from the sterilizer unit 1 itself, such as in a different room. The sterilizer unit 1 has a transmitter 18 that transmits signals to the remote alarm 2, such as via a mains electricity cable 19, or via an optical, ultrasonic or radio link.

## Description

This invention relates to sterilizer apparatus of the kind including a sterilizer unit in which articles to be sterilized can be placed and an alarm for indicating the end of a sterilization cycle.

Sterilizers, such as autoclaves, are used to treat medical and surgical instruments so as to kill bacteria and viruses by the application of heat or radiation. Autoclaves have a pressure chamber into which the item to be treated is placed. Steam at elevated temperature and pressure is supplied to the chamber to sterilize the contents. It is important that the sterilizing cycle is completed before the instruments are removed. For this reason, the door of the sterilizer is locked automatically and cannot be opened until the sterilizing cycle is completed.

The time taken to sterilize instruments can be a considerable problem especially, for example, in small dental surgeries where there may not be sufficient stocks of some instruments. This can mean that surgery has to be delayed until sterilization has been completed. In many surgeries, the sterilizer is not in the surgery itself but in an adjoining anteroom. This is a problem because the dental surgeon or nurse has to go out of the surgery to check the sterilizer repeatedly to see if the sterilization cycle has been completed. This can be very wasteful of the time of the person involved.

It is an object of the present invention to provide improved sterilizer apparatus.

According to one aspect of the present invention there is provided sterilizer apparatus of the above-specified kind, characterised in that the alarm is a unit located remote from the sterilizer unit, that the sterilizer unit includes a transmitter that transmits a signal indicative of the end of a sterilization cycle, and that the alarm unit includes a receiver that receives the signal and provides an indication of the end of the sterilizing cycle at a location remote from the sterilizer unit.

The transmitter may also transmit a signal indicative of a fault in the sterilizer unit, the alarm unit providing an indication of the fault. The transmitter may be connected to a mains electrical cable and transmit the signal along the cable to a mains power circuit, the alarm unit being connected to the mains power circuit so that it receives the signal via the circuit. Alternatively, the transmitter may include an optical emitter, the alarm unit including an optical receiver that receives optical signals from the transmitter. In another arrangement, the transmitter may include an acoustic emitter, the alarm unit including an acoustic receiver that receives acoustic signals from the transmitter. The transmitter may alternatively include a radio transmitter, the alarm unit including a radio receiver that receives radio signals from the transmitter. The alarm preferably includes an audible alarm or a visual display operated when the signal is received from the sterilizer unit.

Sterilizer apparatus in accordance with the present invention, will now be described, by way of example, with reference to the accompanying drawings, in which:
- Figure 1: shows schematically one embodiment of the apparatus; and
- Figures 2 to 4: show alternative embodiments of the apparatus.

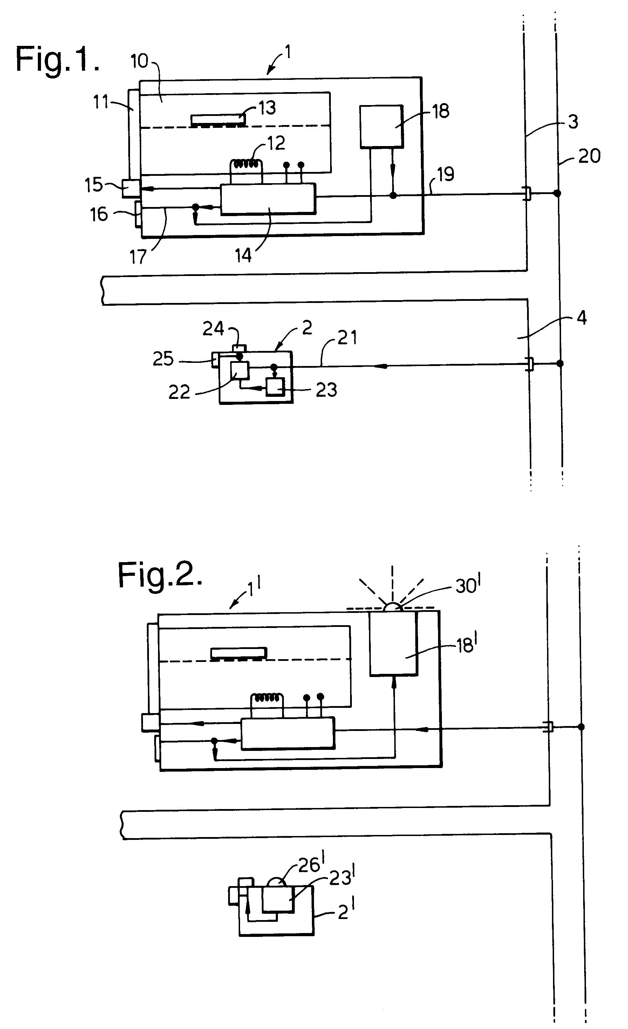

With reference to Figure 1, the sterilizer apparatus includes a mains-operated sterilizer unit 1 and a remote unit 2 located in different rooms 3 and 4.

The sterilizer unit 1 has a pressure chamber 10 that is closed by a door 11. A heating element 12 inside or outside the chamber 10 produces steam at elevated temperature and pressure that effectively sterilizes instruments or other articles 13 placed within the chamber. A control unit 14 controls energization of the heating element 12 and provides conventional monitoring of the temperature and pressure in the chamber. The control unit 14 also controls a lock 15 on the door 11 that prevents the door being opened until the sterilization cycle has been completed. The sterilization unit 1 has a small display panel 16, which is connected to the control unit 14 by a line 17. The display 16 indicates when the sterilization cycle is complete and shows any faults, such as failure to reach the operating temperature and pressure. As so far described, the sterilization unit 1 itself is conventional and may be similar to the sterilizers described in GB 2238407, GB 2237131 or GB 2237110.

The sterilization unit 1 differs from previous sterilizers in that it includes a transmitter 18 connected to the line 17 to receive a sample of the output from the control unit 14 to the display 16. The transmitter 18 converts the output on line 17 into a coded form at a frequency greater than that of the mains power supply and supplies the coded signal onto the mains supply cable 19 of the sterilizer unit 1. These coded signals are supplied onto the mains power ring circuit 20 supplying the building in which the unit 1 is installed.

The remote unit 2 also has a mains cable 21 and connects to the mains ring circuit 20 in the room 4 or in any other room in the building. The remote unit 2 has a drive unit 22 and a receiver/decoder 23 that decodes the coded signals on the mains supply and produces an output to the drive unit 22. The remote unit 2 also has an audible alarm 24 that is energized by the drive unit 22 to produce an audible sound when a coded signal is received indicative of the end of the sterilization cycle. A light 25 or other visible display indicates visibly that the sterilization cycle has finished. When the decoder 23 receives a signal indicative that there is a fault on the sterilization unit 1, it controls the drive unit 22 to sound the audible alarm 24 and to produce a different visible display indicative of a fault.

The remote unit 2 enables the dental surgeon, nurse or other user to be notified of when the sterilization cycle has finished even when they are not in the same room as the sterilizer unit. This can result in a considerable saving of time and ensures that the user is immediately aware when the instruments can be removed. If the remote unit also indicates any fault, it enables this to be rectified quickly. Several remote units 2 could be installed in different rooms so that the user is notified wherever he might be.

The sterilizer apparatus could have various different forms of remote alarm unit. In the arrangement of Figure 2, the transmitter 18' in the unit 1' includes an infra-red emitter 30', such as an LED that emits coded infra-red pulses. The remote unit 2' has an infra-red receiver 26' that supplies electrical signals derived from the received infra-red signals to the decoder 23'. This optical arrangement is only suitable where the remote unit 2' is within line-of-sight of the sterilizer unit 1', either directly or by reflection. The advantage of this arrangement, however, is that it can be battery powered and is, therefore, more portable.

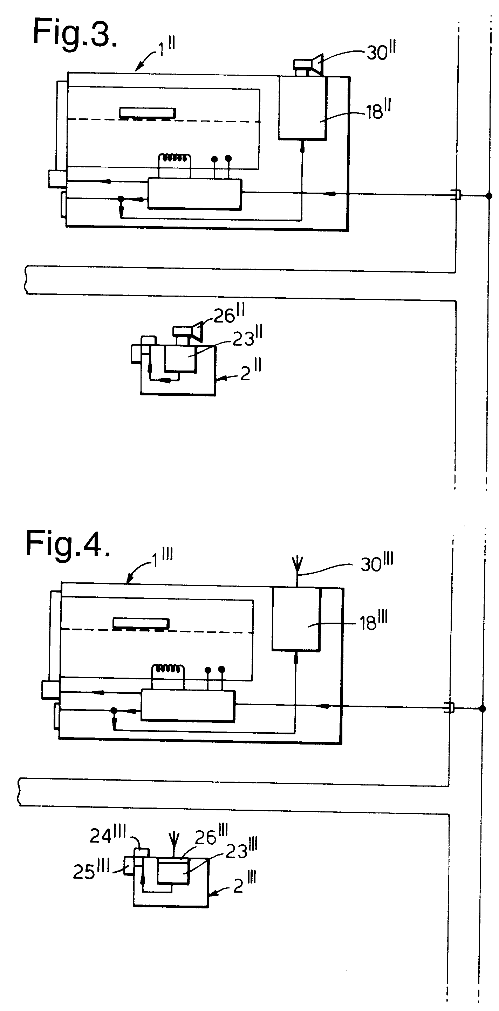

Figure 3 shows an acoustic arrangement where the transmitter 18'' in the unit 1'' includes an ultrasonic emitter 30'' that produces coded ultrasonic signals. The remote unit 2'' has an ultrasonic microphone 26'', or the like, which converts received ultrasonic energy into electrical signals and supplies these to the decoder 23''. This remote alarm unit 2'' need not be used within sight of the sterilizer unit and it can be battery powered and portable. The remote unit 2'' could be carried in a pocket or clipped to the user's clothing.

In the arrangement shown in Figure 4, the transmitter 18''' in the sterilizer unit 1''' is a radio transmitter that transmits short-range radio signals that are received by a receiver 26''' in the remote unit 2'''.

The signal path between the sterilizer unit and the remote alarm unit need not be provided by mains wiring or by a wireless link. Instead, the two units could be connected by a low-voltage cable.

## Claims

1. Sterilizer apparatus including a sterilizer unit (1, 1', 1'', 1''') in which articles (13) to be sterilized can be placed and an alarm (2, 2', 2'', 2''') for indicating the end of a sterilization cycle, characterised in that the alarm is a unit (2, 2', 2'', 2''') located remote from the sterilizer unit (1, 1', 1'', 1'''), that the sterilizer unit includes a transmitter (18, 18', 18'', 18''') that transmits a signal indicative of the end of a sterilization cycle, and that the alarm unit includes a receiver (23, 23', 23'', 23''') that receives the signal and provides an indication of the end of the sterilizing cycle at a location remote from the sterilizer unit.

2. Sterilizer apparatus according to Claim 1, characterised in that the transmitter (18, 18', 18'', 18''') also transmits a signal indicative of a fault in the sterilizer unit (1, 1', 1'', 1'''), and that the alarm unit (2, 2', 2'', 2''') provides an indication of the fault.

3. Sterilizer apparatus according to Claim 1 or 2, characterised in that the transmitter (18) is connected to a mains electrical cable (19) and transmits the signal along the cable to a mains power circuit (20), and that the alarm unit (2) is connected to the mains power circuit (20) so that it receives the signal via the circuit.

4. Sterilizer apparatus according to Claim 1 or 2, characterised in that the transmitter (18') includes an optical emitter (30'), and that the alarm unit (2') includes an optical receiver (23', 26') that receives optical signals from the transmitter (18').

5. Sterilizer apparatus according to Claim 1 or 2, characterised in that the transmitter (18'') includes an acoustic emitter (30''), and that the alarm unit (2'') includes an acoustic receiver (23'', 26'') that receives acoustic signals from the transmitter (18'').

6. Sterilizer apparatus according to Claim 1 or 2, characterised in that the transmitter (18''') includes a radio transmitter (30'''), and that the alarm unit (2''') includes a radio receiver (23''', 26''') that receives radio signals from the transmitter (30''').

7. Sterilizer apparatus according to any one of the preceding claims, characterised in that the alarm unit (2, 2', 2'', 2''') includes an audible alarm (24) that is operated when the signal is received from the sterilizer unit (1, 1', 1'', 1''').

8. Sterilizer apparatus according to any one of the preceding claims, characterised in that the alarm unit (2, 2', 2'', 2''') includes a visual display (25) that is operated when the signal is received from the sterilizer unit (1, 1', 1'', 1''').
